# EUROPEAN PATENT APPLICATION

(11) **EP 1 520 586 A1**
(43) Date of publication of application: **06.04.2005**
(21) Application number: 03292407.8
(22) Date of filing: 30.09.2003
(51) Int. Cl.: A61K 38/17, A61K 39/395, A61P 11/06

(54) **Inhibition of fractalkine or of its receptor for the treatment of atopic allergic diseases**

(71) Applicant: INSTITUT NATIONAL DE LA SANTE ET DE LA RECHERCHE MEDICALE (INSERM), 75013 Paris (FR); UNIVERSITE DE NICE SOPHIA-ANTIPOLIS, 06103 Nice Cédex 02 (FR)
(72) Inventor: Julia, Valérie, 06600 Antibes (FR); Glaichenhaus, Nicolas, 06100 Nice (FR)
(74) Representative: Vialle-Presles, Marie José

(57) **Abstract**

The invention relates to the treatment of atopic allergic diseases in particular respiratory diseases such as allergic rhinitis or asthma, through inhibition of the expression of fractalkine or of its receptor CX3CR1, or through inhibition of their interaction.

## Description

The invention relates to the treatment of any disease which results from the development of an inappropriate T cell-dependent immune response including atopic allergy, aiway inflammation and allergic asthma, through regulation of the interaction between fractalkine (CX3CR1) and its receptor (CX3CR1).

Atopic allergic diseases are characterized by inflammatory reactions resulting from inappropriate IgE antibody response to common environmental antigens. Once IgE antibodies directed against a given allergen have been produced, they bind to mast cells. Later exposure to the same allergen induces mast cells degranulation, and release of numerous pro-inflammatory substances, such as histamine, prostaglandins, leucotrienes, and chemotactic factors for inflammatory cells such as eosinophils.

The clinical expression of atopic allergy depends on the organ wherein the inflammatory reaction takes place. A reaction located to the respiratory tract takes the form of asthma when the lungs are affected, or allergic rhinitis when it is the nose. Allergic rhinitis is often accompanied by allergic conjunctivitis, resulting in so-called "hay fever". A reaction located to the skin induces atopic dermatitis, often under the form of eczema. Food allergy may induce a local reaction in the gastrointestinal tract, and/or distant reactions concerning mainly the skin, as eczema or urticaria, or less frequently, as Quicke oedema. The most dramatic form of atopic allergy is anaphylactic shock, associated with a sudden and generalized allergic reaction which can be fatal if not treated immediately.

Among these diseases, those affecting the respiratory tract and in particular asthma, represent a growing public health problem, since they can be invalidating, and become more and more frequent worldwide.

Atopic allergy is believed to result from an increase in the T helper type 2 (Th2) lymphocytes combined with a decrease in the T helper type 1 (Th1) lymphocytes. Th1 and Th2 cells arise from antigen-stimulated precursor CD4+T cells, that differenciate to either the Th1 or the Th2 phenotype. Although the mechanism of Th1 versus Th2 differentiation is not completely understood, it appears that IL-12 and IL-4 play a major part: IL-12 stimulates the differentiation toward a Th1 phenotype, whereas IL-4 stimulates the differentiation toward a Th2 phenotype.

Th1 and Th2 cells display different profiles of cytokine secretion and are involved in different types of immune response. The Th1 and Th2 cytokines are mutually inhibitory for the functions of the other phenotype.

Th1 cells are characterized by the production of IL-2, IFNγ and TNFβ and are associated with cell-mediated immunity, delayed-type hypersensitivity, macrophage activation, and production of opsonizing antibodies (IgG1 and IgG3). Th2 cells produce in particular IL-4, IL-5, and IL-13, and are associated with humoral response and immediate hypersensitivity, including production of IgE antibodies and recruitment and activation of effector cells such as mast cells and eosinophils.

Recent approaches to treatment of atopic allergic diseases aim at decreasing the Th2 response, by use of Th2 cytokines antagonists, or by correcting the desequilibrium in the Th1/Th2 balance in favour of the Th1 response.

By way of example, in the treatment of asthma, it has been proposed to use cytokines stimulating the Th1 response, such as IL-12 or IFNγ, or inhibitors of cytokines secreted by the Th2 lymphocytes, such as such as IL-4, IL-5, IL-13, and IL-9 (for review, cf. RENAULD, J Clin Pathol, 54:577-89, 2001.

Other therapeutic targets that are currently the subject of many studies are chemokines (chemotactic cytokines), that are involved in the inflammatory response by attracting effector cells.

Chemokines are involved in the control of leukocyte traffic and inflammation. They are characterized by a specific pattern of four conserved cysteine residues. They have been classified into four families, CXC, CX3C, CC and C, dependent on the number and spacing of the first two conserved cysteine residues.

The CXC group includes interleukin 8 (IL-8), MGSA (melanoma growth stimulatory activity) and neutrophil activating peptide 2 (NAP-2); the CC group includes RANTES, MCP-1(monocyte chemotactic protein 1) and MIP-1α (macrophage inflammatory protein 1). The only representatives of the CX3C group and the C groups identified so far are respectively fractalkine, which is chemotactic for T cells and monocytes, and lymphotactin, which is chemotactic for lymphocytes.

The effects of chemokines on leukocytes are mediated by seven-transmembrane domain G protein-coupled receptors. Chemokine receptors are expressed in a different way on different types of leucocytes. Some of them are widely expressed on many leucocytes, while others have a more limited expression. In addition, the expression of chemokine receptors can depend on the cellular activation or the state of differentiation.

For instance, it has been shown that Th1 and Th2 populations have a different chemokine receptor repertoire. Although some chemokine receptors are expressed by both subsets, Th2 cells were reported to express preferentially the receptors CCR3, CCR4, and CCR8, while Th1 cells were reported to express preferentially the chemokine receptors CCR5, CXCR3, and CX3CR1 (BONECCHI et al.. J. Exp. Med., 187, 129-134, 1998; ZINGONI et al.,. J. Immunol., 161, 547-551, 1998; FRATICELLI et al., J. Clin. Invest., 107, 1173-1181, 2001).

Chemokine receptors that are preferentially expressed by Th2 cells, mast cells and/or eosinophils are considered as promising therapeutic targets for allergy. One example is CCR3: this receptor and its ligands (in particular eotaxin) have been shown to play a major part in eosinophils migration and antagonists thereof are attractive candidates for the treatment of atopic allergy.

On the other hand, consistently with their reported role in the amplification of Th1 response (FRATICELLI et al, cited above) fractalkine (CX3CL1) and its receptor (CX3CR1) have been proposed as therapeutic targets for treating Th1-mediated pathologic conditions such as rheumatoid arthritis (PCT WO 01/60406; RUTH et al., Arthritis Rheum. 44, 1568-1581, 2001), or more generally, diseases involving killer lymphocytes (US application 2002/0192212). Fractalkine and CX3CR1 have also been associated with inflammatory conditions such as renal inflammation (COCKWELL et al., J. Pathol. 196, 85-90, 2002 ; CHEN et al., J. Exp. Med., 188, 193-198, 1998), allograft rejection (ROBINSON et al., J. Immunol. 165, 6067-6072, 2000), neurodegenerative disorders, demyelinating diseases and pain (PCT WO 02/076990).

Concerning the involvement of CX3CR1 in airway diseases, it has been reported that G glycoprotein of respiratory syncytial virus (RSV) can bind and activate CX3CR1, and compete with fractalkine for the occupancy of said receptor, resulting in an alteration of Th1 and NK cell responses through interference with fractalkine activity (TRIPP et al., Nat. Immunol., 2, 732-738, 2001; HAYNES, J. Virology, 77, 9831-9844, 2003).

In analyzing the immune response in mice sensitized by *Leishmania* LACK allergen (which induces a Th2 response), the inventors have observed that LACK-specific CD4⁺ T cells generated upon challenge with LACK aerosols expressed higher levels of CX3CR1 chemokine receptor in peri-bronchial lymph nodes (PBLN) than in the spleen. The inventors have then undertaken to investigate the role of CX3CR1 in the migration of LACK-specific CD4⁺ T cells to the airways, using CX3CR1-deficient mice in which both alleles of the CX3CR1 gene have been replaced by the eGFP reporter gene (CX3CR1^{eGFP+/+}mice) . They found that, surprisingly, these CX3CR1-deficient mice were impaired in their ability to develop a Th2 response (and in particular to secrete IL-4, IL-5 and IL-13) upon antigenic restimulation.

These findings allow to propose new means of treatment of atopic allergic diseases by use of fractalkine/CX3CR1 antagonists, or by use of inhibitors of the expression of fractalkine or of CX3CR1.

Inhibitors of the expression of fractalkine or of CX3CR include for instance antisense oligonucleotides interfering RNAs, or ribozymes, targeting the fractalkine gene or the CX3CR1 gene.

A "fractalkine/CX3CR1 antagonist" is herein defined as a compound able to inhibit the activation of CX3CR1 by fractalkine, without being able to activate CX3CR1 on its own. This includes in particular compounds able to interact with fractalkine, and to inhibit its binding with CX3CR1 or to inhibit the activation of CX3CR1 resulting from said binding, as well as compounds able to interact with CX3CR1, and to inhibit its binding with fractalkine or its activation resulting from said binding.

The invention thus proposes the use of an inhibitor of the expression of fractalkine or of CX3CR1 or of a fractalkine/CX3CR1 antagonist for preparing a medicament for treating an atopic allergic disease.

As indicated above, atopic allergic diseases include in particular asthma, allergic rhinitis, allergic conjunctivitis, hay fever, atopic dermatitis, Quicke oedema, and anaphylactic shock.

According to a preferred embodiment of the invention, said atopic allergic disease is an airway disease, in particular asthma or allergic rhinitis.

Examples of fractalkine/CX3CR1 antagonists that can be used according to the present invention include for instance anti-fractalkine antibodies or anti-CX3CR1 antibodies (or antigen-binding fragments thereof), or synthetic peptides selected on their ability to inhibit the activation of CX3CR1 in presence of fractalkine, or the antagonists of CX3CR1 disclosed in PCT WO 02/076990.

Other fractalkine/CX3CR1 antagonists can be identified, for example, by screening a collection of candidate compounds for their ability to inhibit the activation of CX3CR1 in presence of fractalkine. Methods for measuring the activation of G protein-coupled receptors that are known in themselves, and are for instance commonly used in high-throughput screening assays, can be used for evaluating the activation of CX3CR1.

Said fractalkine/CX3CR1 antagonists can be administered by themselves, or mixed with suitable carriers or excipient(s). They can be used systemically or locally. Local administration may be preferred in the case of airway diseases such as asthma or allergic rhinitis, or in the case of cutaneous diseases such as atopic dermatitis. In these cases, one can use any formulation suitable for local administration, such as a solution, a suspension, a gel, an ointment, for administration on the skin, or for instance a spray or an aerosol for administration in the respiratory tract.

The present invention will be further illustrated by the additional description which follows, which refers to non-limitative examples illustrating the effects of the inactivation of CX3CR1 on the immune response to an allergen in mice.

### MATERIALS AND METHODS

### Mice and induction of allergic asthma

CX3CR1^{eGFP+/+} mice on BALB/c background have been previously described (JUNG et al., Mol Cell Biol 20, 4106-4114, 2000). WT15 TCR transgenic mice have been described (WANG et al., J Exp Med, 194, 1721-1730, 2001). Female BALB/c ByJ mice were purchased from Charles River (Lyon, France). CX3CR1^{eGFP+/-} mice were obtained by crossing CX3CR1^{eGFP+/+} mice to BALB/c mice. Mice were raised in our specific pathogen-free animal facility and used between 6 and 8 weeks of age. Sensitization was performed by 2 i.p. injections of 10 µg LACK protein precipitated in 2 mg of aluminum hydroxide (ALUM) (PerBio Science France SAS, Brebieres, France) at day 0 and 7. At day 14, 15, 16, 17 and 18, sensitized mice were exposed to LACK aeorosols (1.5 mg/ml) for 30 minutes. Aerosolization was performed using an Ultramed, ultra-son nebulizator (Medicalia, Italy) connected to a 6500 cm³ box that was used as a deposition chamber.

### Reagents and antibodies

LACK peptide (aa 156 through 173; ICFSPSLEHPIVVSGSWD) was purchased from Mimotopes (Paris, France). LACK recombinant protein was produced in *E. Coli* and purified as described (MOUGNEAU et al., Science *268*, 563-566, 1995). Soluble I-A^{d}/LACK dimers were produced and used as described (JULIA et al., Immunity *16*, 271-283, 2002). Briefly, staining reagent was prepared by incubating 0.7 µl of protein A coupled to the indicated Alexa Fluor® reagent (Molecular Probes Inc., Interchim, Montlugon, France) at the concentration of 0.5 mg/ml in PBS with 4 µg of I-A^{d}/LACK dimers for 30 min at room temperature. The following mAbs were used for cell purification, or flow-cytometry analysis: GK1.5, anti-CD4; 53-6.7, anti-CD8α; M1/170, anti-CD11b; RA3-6B2, anti-B220; AMS-32.1, anti-I-A^{d}; HL3, anti-CD11c; RB6-8C5, anti-Gr-1; 5B11, 2.4G2, anti-FcγIII/II receptor, H1-2F3, anti-CD69. All mAbs were purchased from BD Biosciences (Le Pont de Claix, France).

### Antibody titers

Mice were bled 7 days after the second immunization. LACK-specific IgG1 and IgG2a were measured by ELISA as described (McSORLEY et al., Immunity 7, 401-409, 1997).

### Analysis of bronchoalveolar lavage (BAL) cells

Mice were bled and a canula was inserted into the trachea. Lungs were washed 4 times with 1 ml of warmed PBS. Cells were washed with PBS, resuspended in 200 µl, and counted using a Burker-Turk chamber. For differential BAL cell counts, cytospin preparations were made and stained with Wright/Giemsa. At least 400 cells were scored for each slide, and the numbers of lymphocytes, neutrophils, eosinophils, and macrophage/DC were determined by microscopic examination.

### Purification of cells

For purification of CD4⁺ T cells, LN and spleen cells were depleted of I-A^{d+}, B220⁺, CD11b⁺, CD8α⁺, and Gr-1⁺ cells by negative depletion using sheep anti-rat coated Dynabeads (Dynal, Compiègne, France) as described (JULIA et al., Science 274, 421-423, 1996). For staining with I-A^{d}/LACK multimers, lung T cells were enriched through a Percoll gradient as described (JULIA et al., 2002). Briefly, cell suspensions were depleted of red blood cells, washed and resuspended in 6 ml of 90% Percoll. A 6 ml layer of Percoll 40% was added delicately on the top. After centrifugation at 1000 rpm for 30 minutes, cells at the interface were collected. For purification of spleen and LN I-A^{d}/LACK⁺ and I-A^{d}/LACK⁻ cells, 120 mice that had been previously sensitized and challenged were sacrificed 2 days after the last aerosol. Briefly, spleen and LN CD4⁺ cells were stained with I-A^{d}/LACK multimers, cychrome (CyCR)-conjugated anti-CD4 mAbs, and with a cocktail of phycoerythrin (PE)-conjugated mAbs: anti-B220, anti-CD11c, anti-CD11b, anti-Gr-1 and anti-CD8α mAbs. I-A^{d}/LACK⁺CD4⁺PE⁻ and I-A^{d}/LACK⁻ CD4⁺PE⁻ cells were sorted on a high-speed sorter VANTAGE SETLO⁺ flow cytometer (Becton Dickinson, Le Pont de Claix, France). For purification of lung and BAL I-A^{d}/LACK⁺ and I-A^{d}/LACK- CD4⁺ cells, 300 mice were sacrificed 4 and 7 days after the last aerosol, respectively. To prepare lung cells, lungs were washed 3 times with PBS and cell suspensions were prepared as described below. Cells were depleted from red blood cells, washed, incubated with 2.4G2 mAbs, and stained with CyCR-conjugated anti-CD4 mAbs. CD4⁺ T cells were sorted by FACS, and further stained with I-A^{d}/LACK multimers and the cocktail of lineage-specific PE-conjugated mAbs described above. I-A^{d}/LACK⁺ and I-A^{d}/LACK⁻ CD4⁺ + T cells were sorted by FACS after gating on CD4⁺ PE⁻ cells.

### Flow cytometry

For staining with I-A^{d}/LACK multimers, 10⁶ CD4⁺ T cells were preincubated with 2.4.G2 anti-FcR mAbs, and stained with 4 µg/ml of I-A^{d}/LACK multimers for 30 min at room temperature in PBS supplemented with 0.5% of BSA. Cells were washed and incubated with anti-CD4 mAbs, and with the cocktail of PE-conjugated mAbs described above. Lymphocytes gated by forward- and side-scatter parameters were analyzed on a FACScalibur flow cytometer (Becton Dickinson).

### Cytokine assays

Lungs and PBLN were removed separately, cut into small pieces, and digested with collagenase D (Roche Diagnostics, Meylan, France) for 1 hr at 37°C. Cell suspensions were filtered through a 70 µm cell strainer and depleted of red blood cells using lysis buffer. Cells were washed with Hanks BSS solution (Invitrogen SARL, Cergy-pontoise, France). 5 X 10⁵ cells were incubated for 72 hrs in either 24-well plates for lung cells, or in 96-well plates for PBLN cells, in medium containing either 0.25 mg/ml LACK protein or 20 µM LACK peptide. Supernatants were analyzed for IL-4, IL-5, and IFN-γ.contents by ELISA as described (JULIA et al., 2002). Supernatants were analyzed for IL-13 content by ELISA, using a quantitative mouse IL-13 kit (R&D Systems, Inc., Lille, France). RPMI 1640 containing Glutamax I (Invitrogen), 10% heat-inactivated FCS (Perbio), 0.05 µM 2-mercaptoethanol (Sigma), 2 mM L-glutamine (Invitrogen) and penicillin/streptomycin (Invitrogen), was used as medium for all assays.

### Measurement of chemokine and chemokine receptor mRNA expression

Sorted I-A^{d}/LACK⁺CD4⁺ and I-A^{d}/LACK-CD4⁺ cells were frozen in RNeasy® lysis buffer (Qiagen Inc.). Total RNA was isolated using the RNeasy® 96 kit (Qiagen Inc.) according to the manufacturer's instructions. RNA was transcribed into cDNA by incubation with random hexamers (Promega Corporation, Madison, WI) and oligo (dT) ₁₂₋₁₈ (Pharmacia Biotech Inc., Piscataway, NJ), followed by incubation for 50 min at 45°C in 100 µl containing superscript II reverse transcriptase (Gibco BRL), acetylated BSA (Gibco BRL), dNTPs (Amersham Pharmacia Biotech AB), DTT (Gibco BRL), rRNasin® (Promega), and 1X synthesis buffer (Gibco BRL). Samples were incubated for 45 min at 37°C with ribonuclease H (Gibco BRL) and the samples were stored at -20°C until later use. Real-time quantitative PCR was performed using the ABI Prism® 7700 sequence detection system (Perkin Elmer Applied Biosystems, Foster City, CA) as described (SCHEERENS et al., Eur J Immunol 31, 1465-1474, 2001). Sense and antisense primers, and probes were pre-developed TaqMan® assay reagents (Perkin Elmer Applied Biosystems). 18S rRNA was used as an internal control to measure the amount of RNA in each sample. Data were expressed in arbitrary units normalized to the expression of ubiquitin mRNA.

### RESULTS

### EXAMPLE 1: CHEMOKINE RECEPTOR mRNA EXPRESSION IN ALLERGEN-SPECIFIC T CELLS

To measure the level of expression of chemokine receptors mRNA in allergen-specific CD4⁺ T cells, BALB/c mice were sensitized and challenged with LACK, and sacrificed 4 days after the last aerosol. PBLN, lungs, BAL and spleen cells were harvested and both I-A^{d}/LACK⁺ and I-A^{d}/LACK⁻ CD4⁺ T cells were purified by flow cytometry after staining with I-A^{d}/LACK multimers. The purified cells were used to prepare RNA and levels of chemokine receptors mRNA were measured using quantitative real time PCR using pre-developed TaqMan® assay reagents.

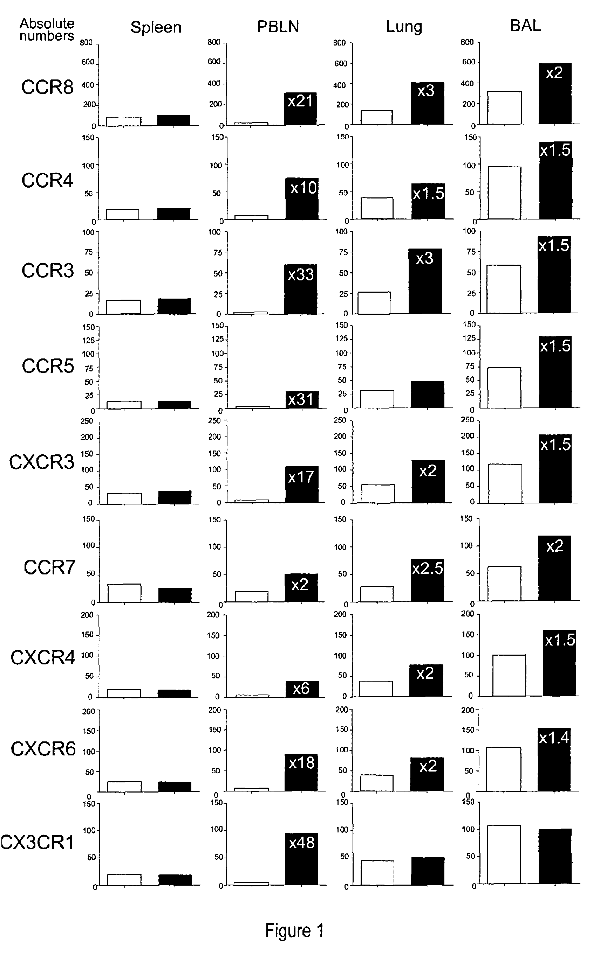

The results are shown in Figure 1.

Legend of figure 1: RNA expression is shown for the indicated chemokine receptors in I-A^{d}/LACK⁺ (filled bars) and I-A^{d}/LACK⁻ (empty bars) CD4⁺ T cells. Data are expressed as arbitrary units after normalization to the level of ubiquitin mRNA. Numbers indicate the ration between mRNA expression in I-A^{d}/LACK⁺ as compared to I-A^{d}/LACK⁻ cells.

These results show that all tested chemokine receptors mRNA were expressed at the same levels in spleen I-A^{d}/LACK⁺ and LACK⁻ CD4⁺ T cells. In contrast, I-A^{d}/LACK⁺ CD4⁺ T cells purified from PBLN contained 10-30 fold more CCR3, CCR4, CCR8, CCR5, CXCR3 and CXCR6 mRNA than PBLN I-A^{d}/LACK⁻ CD4⁺ T cells. PBLN I-A^{d}/LACK⁺ CD4⁺ T cells also expressed 50 fold more CX3CR1 mRNA than I-A^{d}/LACK⁻ CD4⁺ T cells. As compared to PBLN I-A ^{d}/LACK⁻ CD4⁺ T cells, I-A ^{d}/LACK⁺ CD4⁺ T cells also expressed 2 and 6 fold more CCR7 and CXCR4 mRNA respectively.

Lung and BAL I-A^{d}/LACK⁺ CD4⁺ T cells expressed similar amounts of CX3CR1 mRNA, and 2-3 fold more CCR3, CCR4, CCR8, CCR7, CXCR4, CXCR3 and CXCR6 mRNA than I-A^{d}/LACK⁻ CD4⁺ T cells purified from the same compartments. Also, for each chemokine receptor and for both I-A^{d}/LACK⁺ and I-A^{d}/LACK⁻ CD4⁺ T cells, the amount of chemokine receptor mRNA was higher in airway associated tissues as compared to spleen.

### EXAMPLE 2: I-A^{D}/LACK⁺ CD4⁺ T CELLS EXPRESS CX3CR1 IN BAL AND LUNG

To further investigate the expression of CX3CR1 at the surface of allergen-specific T cells, CX3CR1-deficient CX3CR1^{eGFP+/+} mice in which both alleles of the CX3CR1 gene have been replaced by the eGFP reporter gene, were crossed with BALB/c mice, resulting in CX3CR1^{eGFP+/-} mice which carried one copy of the eGFP reporter gene and one copy of the CX3CR1 wt gene. Syngenic BALB/c mice were used as controls.

Mice (5 mice per group) were sensitized and challenged with LACK and sacrificed 1 day after the last aerosol. CD4⁺ T cells were partially purified from blood, spleen, PBLN, lungs, and BAL, and analyzed by FACS after staining with Alexa-Fluor®⁻⁶³³ conjugated protein A bound I-A^{d}/LACK dimers and PerCP-anti-CD4 mAb.

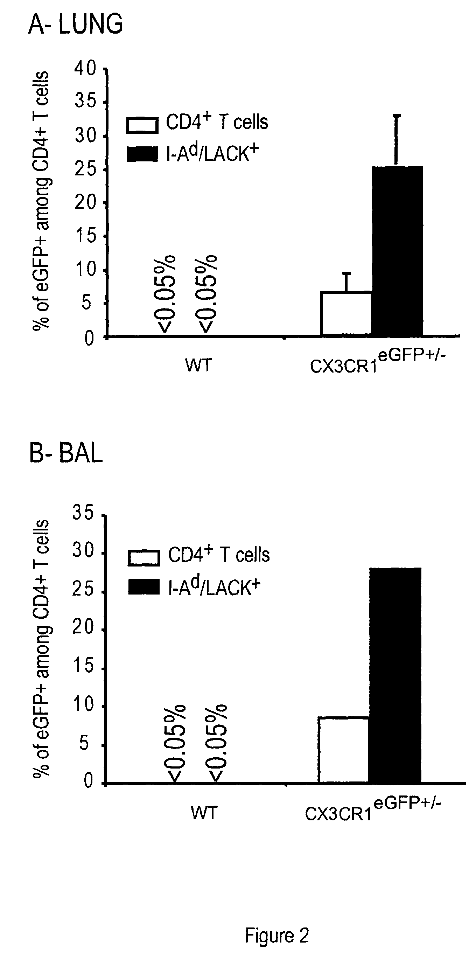

Figure 2 shows the frequency of eGFP⁺ cells among CD4⁺ T cells after gating on lymphocytes, in lung (A) and BAL (B).

As expected, eGFP⁺ CD4⁺ T cells were not detected in wt BALB/c mice. In contrast, 5-10% of total CD4⁺ T cells and 20-30% of I-A^{d}/LACK⁺ cells were eGFP⁺ respectively, in the lungs and BAL of CX3CR1^{eGFP+/-} mice. eGFP⁺ CD4⁺ T cells were not detected in blood, spleen and PBLN (data not shown).

### EXAMPLE 3: LACK-SENSITIZED CX3CR1^{ECFP+/+} MICE DO NOT EXHIBIT AIRWAY EOSINOPHILIA UPON CHALLENGE WITH LACK AEROSOLS

To investigate the role of CX3CR1 in allergic airway inflammation, CX3CR1-deficient CX3CR1^{eGFP+/+} and control wt BALB/c mice (5 mice per group) were sensitized and challenged or not with LACK and sacrificed 1 day after the last aerosol. BAL cells were harvested from individual mouse, centrifugated onto a glass slide using a cystospin centrifuge, and analyzed for cellular contents by optical microscopy after Wright/Giemsa coloration.

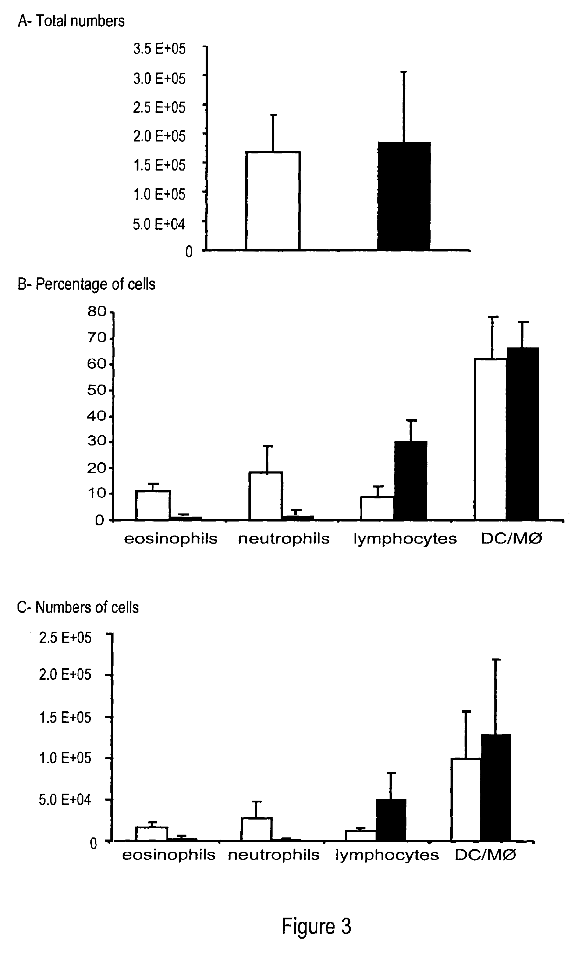

Figure 3 shows (A) the total number of cells recovered from BAL, (B) the frequency of each cell type among total cells and (C) the number of each cell types in BAL.

As previously observed, the numbers of lymphocytes, DC, macrophages eosinophils and neutrophils were higher in wt mice upon challenge with LACK (JULIA et al., 2002). LACK aerosols induced airway cellular infiltration in both CX3CR1^{eGFP+/+} mice and wt mice. However, in contrast to wt mice, BAL from CX3CR1^{eGFP+/+} mice contained almost no eosinophils and neutrophils. Furthermore, BAL from CX3CR1^{eGFP+/+} mice contained 4-fold more lymphocytes than those from wt mice.

### EXAMPLE 4: T CELLS MIGRATION TO THE LUNGS IS IMPAIRED IN CX3CR1^{EGFP+/+} MICE

To further investigate the role of CX3CR1 in the migration of T cells to the airways, CX3CR1^{eGFP+/+}, CX3CR1^{eGFP+/-} and wt mice (5 mice per group) were sensitized and challenged with LACK. Mice were sacrificed 1 day after the last aerosol and PBLN, lung, and BAL cells were harvested from individual mouse. PBLN and lung cell suspensions were analyzed individually and BAL cells were pooled. Cells were analyzed by FACS after staining with Alexa Fluor⁶³³-conjugated protein A bound I-A^{d}/LACK dimers and anti-CD4 mAb.

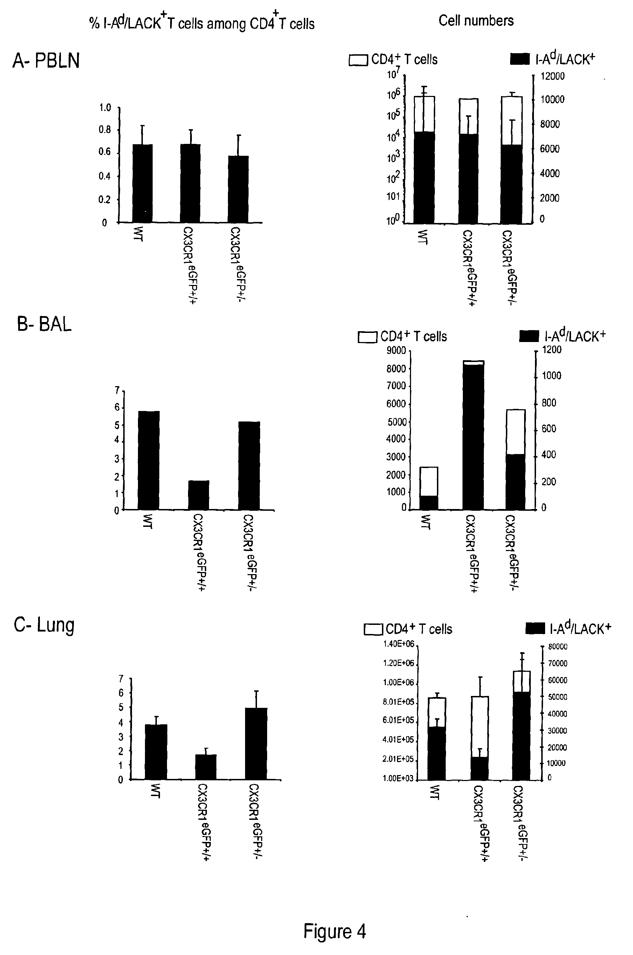

Figure 4 shows the frequency of I-A^{d}/LACK⁺ cells among CD4⁺ T cells in (A) PBLN, (B) BAL and (C) lung cells, after gating on lymphocytes (left panels) and the numbers of total CD4⁺ T cells (empty bars) and of I-A^{d}/LACK⁺CD4⁺ T cells (filled bars) (right panels).

The frequency and number of I-A^{d}/LACK⁺ CD4⁺ T cells in PBLN were similar in CX3CR1^{eGFP+/+} , CX3CR1^{eGFP+/-} and wt mice (Figure 4A) . In contrast, BAL from CX3CR1^{eGFP+/+} mice contained 3 and 10-12 fold more I-A^{d}/LACK⁺ CD4⁺ T cells than those from CX3CR1^{eGFP+/-} and wt mice respectively (Figure 4B). Furthermore, whereas I-A^{d}/LACK⁺ cells accounted for 3.8 +/- 0.6 and 4.8 +/- 1.2 % of lung CD4⁺ in wt and CX3CR1^{eGFP+/-} mice respectively, they represented less than 1.6 +/- 0.5 % of CD4⁺ T cells in CX3CR1^{eGFP+/+} mice (Figure 4C). Accordingly, the lungs of CX3CR1^{eGFP+/+} mice contained 4-5 and 2-3 fold less I-A^{d}/LACK⁺ CD4⁺ T cells than those from CX3CR1^{eGFP+/-} and wt mice respectively (Figure 4C).

### EXAMPLE 5: T CELL RESPONSES ARE IMPAIRED IN THE AIRWAYS OF CX3CR1^{EGFP+/+} MICE

To further characterize T cells in CX3CR^{1eGFP+/+} mice, WT, CX3CR1^{eGFP+/+}, and CX3CR1^{eGFP+/-} mice were sensitized and challenged with LACK, and sacrificed 1 day after the last aerosol. PBLN and lung cells were harvested and incubated *in vitro* with or without LACK peptide for 72 hours. Supernatants were harvested and analyzed for cytokine (IL-4, IL-13, IL-5 and IFN-γ)content by ELISA.

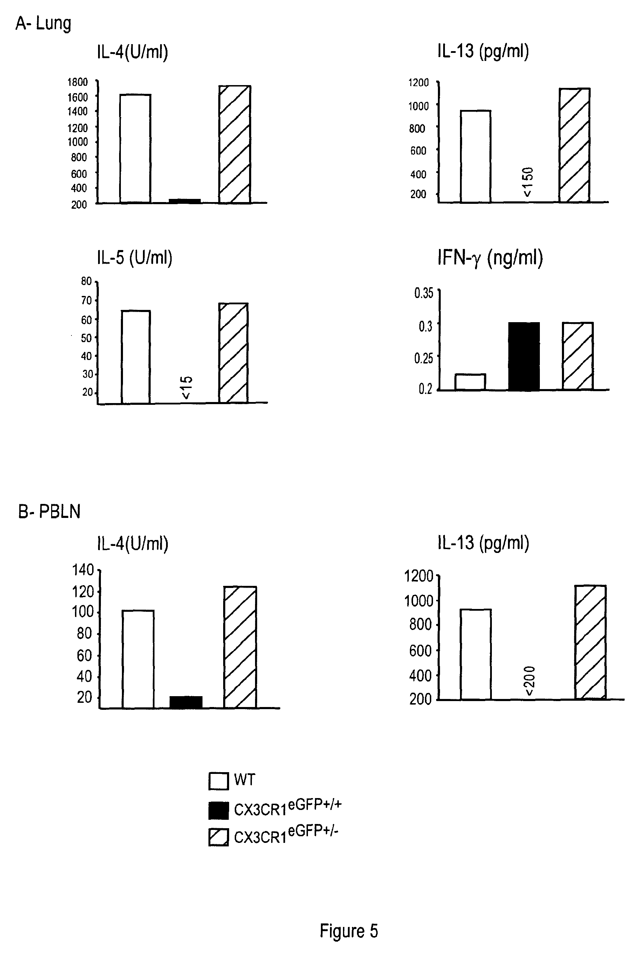

The results are shown in Figure 5.

Legend of figure 5: (A) Lung cells; (B) PBLN cells from WT (empty bars), CX3CR1^{eGFP+/+} (filled bars), and CX3CR1^{eGFP+/-} (dashed bars) mice were sensitized and challenged with LACK, and sacrificed 1 day after the last aerosol. were harvested and incubated with or without LACK peptide for 72 hours. Cytokine contents were measured by ELISA. Data show the amount of IL-4, IL-13, IL-5 and IFN-γ upon restimulation with LACK. None of these cytokines was detected when cells were incubated without LACK (data not shown). The limit of detection for IL-4, IL-13, IL-5 and IFN-γ were 200 U/ml, 150 pg/ml, 15 U/ml and 0.2 ng/ml respectively. IFN-γ was below the level of detection in all PBLN samples.

While both lung and PBLN cells from CX3CR1^{eGFP+/-} and wt mice secreted detectable amounts of IL-4, IL-5 and IL-13 in response to LACK, these cytokines were not detectable in the supernatants of cells from CX3CR1^{eGFP+/+} mice (Figure 5A). In contrast, lung cells from all mice secreted similar low levels of IFN-γ in response to LACK (Figure 5A) . Thus, lung T cells from CX3CR1^{eGFP+/+} mice were selectively impaired in their ability to secrete Th2 cytokines upon antigenic restimulation. This phenomenon was unlikey to result from a difference in T cell priming because sera from CX3CR1^{eGFP+/+}, CX3CR1^{eGFP+/-} and wt mice exhibited similar titers of LACK-specific IgG1 and IgG2a (data not shown).

## Claims

1. Use of a fractalkine/CX3CR1 antagonist or of an inhibitor of the expression of fractalkine or of CX3CR1, for preparing a medicament for treating an atopic allergic disease.

2. Use according to claim 1, wherein said atopic allergic disease is a respiratory disease.

3. Use according to claim 2, wherein said disease is asthma

4. Use according to claim 2 wherein said disease is allergic rhinitis.
